(19) Europäisches Patentamt
European Patent Office
Office européen des brevets

(11) Veröffentlichungsnummer : **0 412 387 B1**

(12) **EUROPÄISCHE PATENTSCHRIFT**

(45) Veröffentlichungstag der Patentschrift : **24.11.93 Patentblatt 93/47**

(21) Anmeldenummer : **90114572.2**

(22) Anmeldetag : **30.07.90**

(51) Int. Cl.$^5$ : **C07C 317/14,** C07C 317/22, C07C 315/04, C07C 321/28, C07C 319/20, A61K 31/10, A61K 7/40

(54) **Diarylacetylene, ihre Herstellung und Verwendung.**

Die Akte enthält technische Angaben, die nach dem Eingang der Anmeldung eingereicht wurden und die nicht in dieser Patentschrift enthalten sind.

(30) Priorität : **08.08.89 DE 3926148**

(43) Veröffentlichungstag der Anmeldung : **13.02.91 Patentblatt 91/07**

(45) Bekanntmachung des Hinweises auf die Patenterteilung : **24.11.93 Patentblatt 93/47**

(84) Benannte Vertragsstaaten : **AT BE CH DE DK FR GB IT LI NL SE**

(56) Entgegenhaltungen : **EP-A- 0 002 742**

(56) Entgegenhaltungen :
**EP-A- 0 084 667**
**EP-A- 0 176 034**
**DE-A- 3 905 932**
**JOURNAL OF THE AMERICAN CHEMICAL SOCIETY, vol. 93, no. 12, 16. Juni 1971, GASTON, PA, US, Seiten 2974 - 2981; J. J. EISCH et al: "POLAR AND STEREOCHEMICAL EFFECTS IN THE ADDITION OF TRIPHENYLALUMINUM TO PARA-SUBSTITUTED DIPHENYLACETYLENES"**

(73) Patentinhaber : **BASF Aktiengesellschaft**
**Carl-Bosch-Strasse 38**
**D-67063 Ludwigshafen (DE)**

(72) Erfinder : **Janssen, Bernd, Dr., Dipl.-Chem.**
**Leuschnerstrasse 18A**
**D-6700 Ludwigshafen (DE)**
Erfinder : **Wuest, Hans-Heiner, Dr., Dipl.-Chem.**
**Unteres Bieth 10**
**D-6915 Dossenheim (DE)**

**EP 0 412 387 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue diarylsubstituierte Acetylene, Verfahren zu deren Herstellung sowie deren Verwendung bei der Prophylaxe und Bekämpfung von Krankheiten.

Aus DE-OS 28 54 354 und DE-OS 32 02 118 ist bekannt, daß retinoidale Benzoesäurederivate pharmakologische Wirkungen bei der topischen und systemischen Therapie von Neoplasien und Dermatosen, z.B. Akne oder Psoriasis, aufweisen. Nachteil dieser Verbindungen ist ihre geringe therapeutische Breite bezüglich der unter dem Begriff Hypervitaminose A zusammengefaßten Nebenwirkungen.

Ferner ist bekannt, daß Acetylenderivate, wie in DE-OS 34 34 946 beschrieben, retinoidale Wirkungen aufweisen, die allerdings nicht immer befriedigen.

Überraschenderweise wurde nun gefunden, daß Diarylacetylene der Formel I

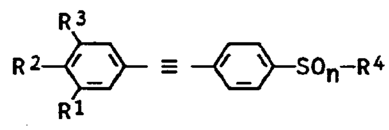

I

in der $R^1$ Wasserstoff oder eine $C_1$-$C_4$-Alkylgruppe bedeutet, $R^2$ und $R^3$ je ein Wasserstoffatom oder - wenn $R^1$ in der Bedeutung von Wasserstoff steht - unter Bildung eines Zyklus gemeinsam eine $-C(CH_3)_2-B-C(CH_3)_2-$Gruppe (mit B in der Bedeutung von $-CH_2CH_2-$oder $-CH(CH_3)-$) oder eine $-C(CH_3)(Z)CH_2CH_2O-$Gruppe (mit Z in der Bedeutung einer $C_1$-$C_2$-Alkylgruppe) darstellen oder
$R^2$ eine $C_1$-$C_3$-Alkoxygruppe und $R^3$ eine $C_1$-$C_{10}$-Alkylgruppe, die geradkettig, verzweigt oder (poly-)cyclisch sein kann, bedeuten oder - wenn $R^2$ für ein Wasserstoffatom steht - $R^1$ und $R^3$ Alkylreste in der oben angegebenen Bedeutung darstellen,
$R^4$ für einen $C_1$-$C_6$-Alkylrest steht und n gleich 0, 1 oder 2 ist, ein breites Wirkspektrum besitzen.

Von den Verbindungen der Formel I sind diejenigen bevorzugt, in denen $R^1$ und $R^3$ verzweigte Alkylgruppen bedeuten, ferner werden Verbindungen bevorzugt, in denen $R^2$ und $R^3$ über eine gemeinsame Gruppe $-C(CH_3)_2-B-C(CH_3)_2-$ einen Zyklus ausbilden, wobei B in der Bedeutung einer $-CH_2CH_2-$Gruppe besonders bevorzugt wird.

Des weiteren werden Verbindungen der Formel I bevorzugt, worin $R^4$ an einen Sulfinyl-(n=1) oder Sulfonyl-(n=2) Rest gebunden ist; der Sulfonrest wird besonders bevorzugt.

Die erfindungsgemäßen Verbindungen kann man herstellen, indem man

a) Stilbene der Formel II

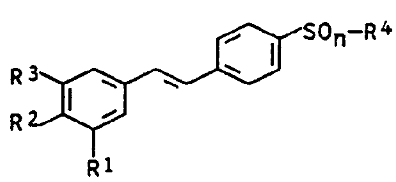

II

in der $R^1$ bis $R^4$ die oben angegebene Bedeutung haben, halogeniert und anschließend 2 Mole Halogenwasserstoff abspaltet, oder

b) ein α-Chlorbenzylphosphonat der Formel III,

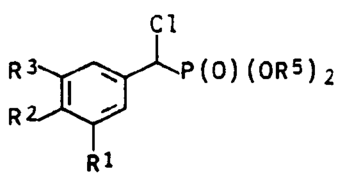

III

in der $R^1$ bis $R^3$ die oben angegebene Bedeutung haben und $R^5$ eine $C_{1-3}$-Alkylgruppe bedeutet, mit Aldehyden der Formel IV,

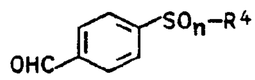 .

IV

mit $R^4$ in der oben angegebenen Bedeutung umsetzt, oder

c) ein α-Chlorbenzylphosphonat der Formel V,

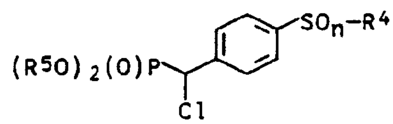

V

in der $R^4$ und $R^5$ die oben angegebene Bedeutung haben, mit Aldehyden der Formel VI

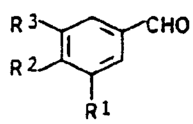

VI

worin $R^1$ bis $R^3$ die oben angegebene Bedeutung haben, umsetzt, oder

d) Monoarylacetylene der Formel VII,

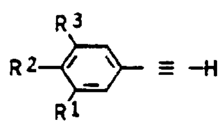

VII

worin $R^1$ bis $R^3$ die oben angegebene Bedeutung haben, mit einem Arylhalogenid der Formel VIII

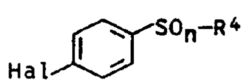

VIII

worin $R^4$ die oben angegebene Bedeutung zukommt und Hal Chlor, Brom oder Iod darstellt, in Gegenwart eines Katalysators und einer Base umsetzt, oder

e) Arylhalogenide der Formel IX

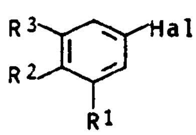

IX

worin $R^1$ bis $R^3$ und Hal die oben angegebene Bedeutung zukommt, mit einem Monoarylacetylen der Formel X

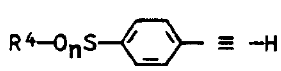

X

worin $R^4$ die oben angegebene Bedeutung besitzt, in Gegenwart eines Katalysators und einer Base umsetzt

und die so erhaltenen Verbindungen gegebenenfalls nach Standardmethoden in weitere Verbindungen der Formel I umwandelt.

Die Halogenierung von Verbindungen der Formel II gemäß a) erfolgt zweckmäßigerweise mit Brom in einem Lösungsmittel bei Temperaturen bis zu 50°C, vorzugsweise im Bereich von -15 bis 0°C. Als Lösungsmittel finden chlorierte Kohlenwasserstoffe, insbesondere Chloroform oder Tetrachlorkohlenstoff Verwendung. Anstelle von freiem Brom können auch Komplexe von molekularem Brom mit Kronethern, z.B. Dibenzo-18-Krone-6 oder Perbromide wie z.B. Tetrabutylammoniumbromid verwendet werden.

Geeignete Basen für die Eliminierung von zwei Moläquivalenten Bromwasserstoff aus den so erhaltenen Dibromverbindungen sind die Hydroxide, Alkoholate, Hydride und Amide der Alkali- und Erdalkalimetalle. Man arbeitet zweckmäßigerweise in einem Lösungsmittel. Man kann auch, falls dies erforderlich ist, unter Ausschluß von Hydroxylionen arbeiten, z.B. mit Kalium-tert.-butanolat als Base in Tetrahydrofuran oder Dimethylsulfoxid bei 25°C bis 60°C, oder besonders vorteilhaft in Petrolether in Gegenwart eines Phasen-Transfer-Katalysators, vorzugsweise 18-Krone-6, beim Siedepunkt des Reaktionsgemisches.

Die Verbindungen der Formel II können nach den in der DE-OS 32 02 125 beschriebenen Verfahren hergestellt werden, zweckmäßigerweiser über Wittig-Olefinierung entsprechender aromatischer Aldehyde mit Phosphonium-Salzen oder Phosphonsäureestern.

Die Umsetzungen nach Wittig-Horner gemäß b) und c) verlaufen bei einer Temperatur bis zu 100°C, zweckmäßig bei 20 bis 50°C. Die Umsetzungen können bei atmosphärischem Druck oder in einem geschlossenen Gefäß unter erhöhtem Druck, gegebenenfalls unter Erwärmung auf den angegebenen Temperaturbereich, durchgeführt werden.

Man kann diese Umsetzungen in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylsulfoxid oder deren Mischungen, wobei die Umsetzung im allgemeinen bei einer Temperatur bis zu 30°C abläuft.

Die Umsetzungen werden in Gegenwart eines Deprotonierungsmittels vorgenommen. Geeignet sind Alkalimetallhydride und Alkalimetallamide, insbesondere des Natriums und Kaliums, die Natrium- und Kaliumsalze von Dimethylsulfoxid, Alkyllithiumverbindungen wie n-Butyllithium oder Alkalimetallalkoholate, vorzugsweise Natriummethanolat, oder Kalium-tert.-butanolat.

Die Gesamtreaktion [b) und c)] ("Wittig-Horner-Reaktion" + Eliminierung) verläuft besonders glatt mit 2 Moläquivalenten Kalium-tert.-butanolat in Dimethylsulfoxid als Lösungsmittel in einem Eintopfverfahren (vgl. J. Amer. Chem. Soc. 87, 2777 (1965)).

Bei Reaktionen d) und e) werden aus Verbindungen der Formel VII, bzw. X in üblicher Weise in situ die entsprechenden Kupferacetylide hergestellt, die mit den Arylhalogeniden VIII bzw. IX, vorzugsweise den Bromiden und -Jodiden, weiter zu Verbindungen der Formel I umgesetzt werden. Alternativ kann die Kupplungsreaktion, direkt von den Acetylenen VII bzw. X ausgehend, durch Triphenylphosphinkomplexe des Palladiums und Nickels katalysiert werden. In allen Fällen ist die Gegenwart einer Base zweckmäßig, beispielsweise einer organischen Stickstoffbase, wie Triethylamin oder Pyridin, oder eines Alkalimetallalkoholats, wie Natriummethanolat oder Natriumphenolat. Gegebenenfalls wird in einem Lösungsmittel gearbeitet, vorzugsweise in Dimethylformamid oder Tetrahydrofuran. Die Umsetzung verläuft bei einer Temperatur von 50 bis 150°C, zweckmäßigerweise bei 50°C (Aryliodide) oder 100°C (Arylbromide).

Vorteilhaft ist gegebenenfalls die Verwendung von Palladiumverbindungen wie Palladiumacetat in Mengen von 0,1 bis 10 Mol%, wobei man die Reaktion in einem basischen Lösungsmittel wie Triethylamin drucklos oder unter erhöhtem Druck bei Temperaturen bis 150°C, vorzugsweise bei der Rückflußtemperatur des Lösungsmittels durchführt (vgl. z.B.: Lambert Brandsma, "Preparative Acetylenic Chemistry", 2nd. Ed., Elsevier, Amsterdam 1988 und dort zitierte Literatur).

Die für die Verfahren b, c, d und e benötigten Ausgangsstoffe sind nach bekannten Verfahren erhältlich:

1-Aryl-1-chlormethylphosphonate der Formel III und der Formel V können z.B. dadurch hergestellt werden, daß man in an sich bekannter Weise den entsprechenden aromatischen Aldehyd mit einem Dialkylphosphit, gegebenenfalls in Gegenwart einer katalytischen Menge Base, z.B. Triethylamin oder Natriummethanolat oder besonders vorteilhaft Kalium-tert.-butanolat umsetzt; die so hergestellten 1-Aryl-1-hydroxymethylphosphonate werden dann üblicherweise mit Thionylchlorid oder Phosphoroxytrichlorid, und falls zweckmäßig, in Gegenwart eines säurebindenden Mittels wie Pyridin oder Triethylamin, behandelt.

Die für die Wittig-Horner-Reaktion benötigten Aldehyde der Formel IV bzw. VI lassen sich beispielsweise durch Formylierung der entsprechenden Benzol-, Tetralin- oder Indanderivate in Gegenwart einer Lewis-Säure herstellen. Die Formylierung wird vorteilhaft mit Hexamethylentetramin/Trifluoressigsäure durchgeführt. Tetrahydrotetramethylnapthalinderivate sind von T.F. Wood et.al. in US-PS 3 442 640 und US-PS 3 499 751

beschrieben oder können nach dem dort angegebenen Verfahren aus 2, 5-Dichlor-2,5-dimethylhexan und einem entsprechend substituierten Benzol durch Friedel-Crafts-Alkylierung hergestellt werden.

Die als Ausgangsprodukte verwendeten Monoarylacetylene der Formel VII lassen sich z.B. wie folgt herstellen:

Ein Arylmethylketon der Formel XI

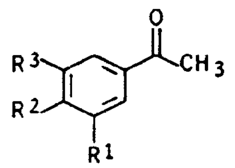

XI

worin $R^1$ bis $R^3$ die genannten Bedeutungen haben, wird in an sich bekannter Weise mit Phosphorpentachlorid in Gegenwart einer Base wie Pyridin bei 0 bis 25°C in das entsprechende 1-Aryl-1-chlor-ethylen umgewandelt, welches mit einer Base, vorzugsweise Kalium-tert.-butanolat, in einem aprotisch dipolaren Lösungsmittel wie Dimethylsulfoxid bei 25 bis 40°C zu dem Monoarylacetylen der Formel VII umgewandelt wird.

Man kann aber auch einen Aldehyd der Formel VI mit einem Phosphoniumsalz der Formel XII,

$(Ar)_3{}^{\oplus}P\text{-}CH_2\text{-}Hal \quad Hal^{\ominus}$ XII

worin Ar für einen aromatischen Rest, vorzugsweise den Phenylrest steht und Hal Chlor, Brom oder Iod, vorzugsweise ein Brom- oder Chloratom bedeutet, in an sich bekannter Weise unter Basenkatalyse im Sinne einer Wittig-Reaktion umsetzen und das so gebildete Vinylhalogenid in situ mit überschüssiger Base zum korrespondierenden Monoacetylen der Formel VII dehydrohalogenieren.

Die Arylhalogenide der Formel VIII sind überwiegend bekannt bzw. lassen sich herstellen nach allgemein bekannten Methoden zur Aromatenhalogenierung [vgl. "Methoden der organischen Chemie" (Houben-Weyl) Bd. 5/3, S. 651 ff, 4. Aufl. 1962; Bd. 5/4 S. 233 ff und 354 ff sowie S. 517 ff 4. Aufl. 1960].

Die Arylhalogenide der Formel IX sind teilweise bekannt oder lassen sich herstellen nach den üblichen Halogenierungsverfahren wie oben angegeben.

Die Acetylene der Formel X sind teilweise neu und werden hergestellt, indem man beispielsweise einen Aldehyd der Formel IV mit einem Phosphoniumsalz der Formel XII entsprechend der Darstellung von Acetylenen VII umsetzt.

Die erfindungsgemäßen Verbindungen der allgemeinen Formel I, in der n gleich 0 ist, können - falls gewünscht - nach üblichen Methoden in die korrespondierenden Sulfoxide bzw. Sulfonderivate umgewandelt werden.

Die Oxidation zu Sulfoxiden erfolgt vorteilhaft, indem man den Thioether in alkoholischer Lösung mit äquimolaren Mengen oder einem bis zu 10-%igen Überschuß von Perjodsäure oder einem Alkalisalz derselben, vorzugsweise mit Natriumsalz, bei Temperaturen von 0 bis 30°C zur Reaktion bringt. Als Löslichkeitsvermittler sind beispielsweise Wasser, Dimethylsulfoxid oder Amide wie Dimethylformamid, aber auch Ketone wie Aceton, geeignet. Die Oxidation zu Sulfonen erfolgt vorteilhaft, indem man 2 bis 3 Äquivalente des Oxidationsmittels bei Temperaturen von -30 bis 120°C, vorzugsweise bei -10 bis 60°C auf den entsprechenden Thioether einwirken läßt. Als Oxidationsmittel eignen sich Wasserstoffperoxid und besonders Peroxicarbonsäuren, worunter m-Chlor-peroxibenzoesäure bevorzugt wird. Als Lösungsmittel werden bei der Verwendung von Wasserstoffperoxid vorzugsweise Essigsäure oder Acetonitril, bei Verwendung von Peroxicarbonsäuren aprotische Lösungsmittel wie Methylenchlorid oder Toluol eingesetzt.

Die neuen Verbindungen der Formel I enthalten teilweise chirale Zentren und fallen im allgemeinen als Diastereomerengemische, bzw. Racemate an. Die so erhaltenen Diastereomeren lassen sich beispielsweise durch Löslichkeitsunterschiede oder durch Säulenchromatographie trennen und in reiner Form isolieren. Aus den Enantiomerenpaaren kann man nach bekannten Methoden einheitliche Enantiomere erhalten. Sowohl diese als auch ihre Gemische (Racemate) werden von der vorliegenden Erfindung umfaßt. Als therapeutische oder kosmetische Mittel kann man sowohl die einheitlichen Diastereomeren bzw. Enantiomeren wie auch deren Gemische verwenden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und inneren Organe sowie bei der topischen und systemischen Therapie von Akne, Psoriasis und anderen mit pathologisch veränderter Verhornung einhergehenden dermatologischen Erkrankungen, insbesondere Ichthyosis, Dariersche Krankheit, Flechte, Leukoplakie, aber auch gegen Vitiligo, Ekzeme, Warzen, Lichtschäden (vorzeitige Alterung) der Haut, ferner gegen trockene Augen und andere Corneopathien sowie zur Behandlung von rheumatischen Erkrankungen,

insbesondere solchen entzündlicher oder degenerativer Art, die Gelenke, Muskeln, Sehnen und andere Teile des Bewegungsapparates befallen, verwendet werden. Bevorzugte Indikationsgebiete sind: Die Therapie von dermatologischen Erkrankungen; die Therapie von durch Sonnenlichteinwirkung bedingte Hautschädigungen; die Therapie von iatrogen, z.B. durch Corticosteroide induzierte Atrophie, bedingten Hautschädigungen und die prophylaktische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1972) oder aus Nature 250, 64-66 (1974) und Nature 253, 47-50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15-22 (1978) und Proc. Natl. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al. in The Journal of Investigative Dermatology 73, 354-358 (1978) beschrieben. Als weiteres Maß für die dermatologische Aktivität kann die Reduktion der Talgdrüsen und die damit einhergehende verminderte Talgproduktion am Seitenorgan des Hamsters dienen. Diese Methodik ist von E.C. Gomez in J. Am. Acad. Dermatol. 6, 746-750 (1982) beschrieben.

Ferner kann die durch die erfindungsgemäßen Verbindungen erzielbare Reversion von Hautschäden, welche durch UV-Licht ausgelöst wurden, in Tiermodellen bestimmt werden. Diese Methodik ist von L.H. Kligman et al. beschrieben in Connect. Tissue Res. 12, 139-150 (1984) und im Journal of the American Academy of Dermatology 15, 779-785 (1986).

Dementsprechend sind ein weiterer Gegenstand der Erfindung kosmetische und therapeutische Mittel zur topischen und systemischen Anwendung, die eine Verbindung der Formel (I) neben üblichen Trägerstoffen oder Verdünnungsmitteln enthalten.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Emulsionen und Sprays.

Die therapeutischen oder kosmetischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung als therapeutische Mittel vorzugsweise in einer Einzeldosis von 0,1 bis 250 mg enthalten und täglich in einer oder mehreren Dosierungen je nach Art und Schwere der Erkrankungen verabreicht werden.

Die Arzneimittel und Kosmetika der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetat phthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Zweckmäßige übliche Bestandteile von kosmetischen und pharmazeutischen Zubereitungen für die topische Anwendung sind beispielsweise: anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere, feste oder flüssige Ölkomponenten bzw. Fettstoffe mineralischer, pflanzlicher oder tierischer Herkunft, synthetische Esteröle, wie Triglyceridester und Isopropylmyristat, hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, ätherische Öle und Parfümöle, Vitamine, Pflanzenextrakte, Collagen usw. Diese Stoffe können beispielsweise dem CFTA, Cosmetic Ingredient Dictionary, 3. Auflage, Washington 1982, entnommen werden.

Die folgenden Beispiele und Vorschriften erläutern die Herstellung der neuen Verbindungen und ihrer Vorprodukte:

Herstellung der Ausgangsverbindungen

Beispiel A

3,5-Di-tert.-butyl-phenylacetylen

117,6 g (0,269 mol) Brommethyltriphenylphosponiumbromid wurden in 420 ml trockenem Tetrahydrofuran unter Kühlung bei 25°C mit 73,7 g (0,655 mol) Kalium-tert.-butanolat portionsweise versetzt. Das Reaktionsgemisch wurde eine halbe Stunde nachgerührt und bei -70°C mit einer Lösung von 50 g (0,218 mol) 3,5-Di-tert.-butyl-benzaldehyd in 120 ml trockenem Tetrahydrofuran tropfenweise versetzt. Nach einer halben Stunde ließ man die Reaktion auf Raumtemperatur erwärmen, rührte über Nacht nach und trug den Ansatz in Wasser ein. Extraktion mit Ether, Waschen der organischen Phase mit Wasser, Trocknen über Natriumsulfat und Abdampfen des Lösungsmittels lieferte einen zähen Rückstand.

Nach Destillation erhielt man 18,7 g der Titelverbindung

$Kp_{0,2}$:90-94°C

$^1H$-NMR ($CDCl_3$): $\delta$ = 1,3(s,18H); 3,05(s,H); 7,4(m,3H)

Beispiel B

4-Methylsulfonyl-brombenzol

40,6 g (0,2 mol) 4-Bromthioanisol wurden in 300 ml Eisessig bei 50°C binnen einer Stunde mit 102,4 ml Wasserstoffperoxid (30 %ig) versetzt. Nach beendeter Reaktion wurde die Lösung auf Eiswasser gegossen, der Niederschlag abgesaugt, mit Wasser gewaschen und im Vakuum getrocknet.

Man erhielt 42 g der Titelverbindung Fp. 102-104°C.

Synthese der Produkte

Beispiel 1

2-(5,6,7,8-Tetrahydro-5,5,8,8-tetramethyl-2-napthyl)-1-(4-methylsulfonylphenyl)-acetylen

3,2 g (0,015 mol) 5,6,7,8-Tetrahydro-5,5,8,8-tetramethyl-2-naphthylacetylen, 3 g (0,0128 mol) 4-Methylsulfonyl-brombenzol, 12,7 g (0,126 mol) Triethylamin, 12,3 mg (0,047 mmol) Triphenylphosphin, 1,9 mg (0,0027 mmol) Bis-(triphenylphosphin)-palladium(II)-chlorid und 6,5 mg (0,034 mmol) Kupfer (I)-jodid wurden in 4 ml trockenem Dimethylformamid unter Stickstoff zum Rückfluß erhitzt. Nach beendeter Reaktion wurde das erkaltete Reaktionsgemisch auf Wasser gegossen mit verdünnter Salzsäure auf pH 5 gestellt und mit Ether extrahiert. Die mit Wasser gewaschene, über Natriumsulfat getrocknete organische Phase ergab nach Abdampfen des Lösungsmittels 5,5 g eines Rückstandes, der nach Umkristallisieren aus Methanol 3,5 g der Titelverbindung lieferte. Fp.: 153-155°C.

Analog zu der in Beispiel 1 beschriebenen Arbeitsweise erhielt man

Beispiel 2

aus 5,6,7,8-Tetrahydro-5,5,8,8-tetramethyl-2-naphthylacetylen und

4-Ethylsulfonyl-brombenzol
2-(5,6,7,8-Tetrahydro-5,5,8,8-tetramethyl-2-naphthyl)-1-(4-ethyl-sulfonylphenyl)-acetylen; Fp.: 166-168°C,

Beispiel 3

aus 5,6,7,8-Tetrahydro-5,5,8,8-tetramethyl-2-naphthylacetylen und
4-Isopropylsulfonyl-brombenzol
2-(5,6,7,8-Tetrahydro-5,5,8,8-tetramethyl-2-naphthyl)-1-(4-iso-propylsulfonylphenyl)-acetylen; Fp.: 152-153°C,

Beispiel 4

aus 3,5-Di-tert.-butyl-phenylacetylen und
4-Ethylsulfonyl-brombenzol
1-(3,5-Di-tert.-butylphenyl)-2-(4-ethylsulfonylphenyl)-acetylen; Fp.: 136-137°C.

Beispiel 5

2-(5,6,7,8-Tetrahydro-5,5,8,8-tetramethyl-2-naphthyl)-1-(4-methylthiophenyl)-acetylen

Zu einer Lösung von 3,2 g (15,8 mmol) 4-Bromthioanisol, 35,5 mg (0,15 mmol) Palladium (II)-acetat, 1,7 g (6,5 mmol) Triphenylphosphin und 120 mg (0,6 mmol) Kupfer(I)-jodid in 80 ml Triethylamin wurden in der Siedehitze 4,0 g (18,9 mmol) 5,6,7,8-Tetrahydro-5,5,8,8-tetramethyl-2-naphthylacetylen, gelöst in 40 ml Triethylamin, zugetropft. Nach beendeter Reaktion wurde der Niederschlag abgetrennt, das Filtrat eingedampft und der Rückstand mit Heptan digeriert.

Man erhielt 1,0 g der Titelverbindung; Fp.: 120-123°C.

Beispiele für pharmazeutische Zubereitungen:

Beispiel I

Tablette mit 250 mg Wirkstoff
Zusammensetzung für 1000 Tabletten:

| | |
|---|---|
| Wirkstoff des Beispiels Nr. 2: | 250 g |
| Kartoffelstärke: | 100 g |
| Milchzucker: | 50 g |
| Gelatinelösung 4 %: | 45 g |
| Talkum: | 10 g |

Herstellung:

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel II

Creme aus 0,1 % Wirkstoff

| | |
|---|---|
| Wirkstoff des Beispiels Nr. 7: | 0,1 g |
| Glycerinmonostearat: | 10,0 g |
| Cetylalkohol: | 4,0 g |
| Polyethylenglykol-400-stearat: | 10,0 g |
| Polyethylenglykol-sorbitanmonostearat: | 10,0 g |
| Propylenglykol: | 6,0 g |
| p-Hydroxybenzoesäuremethylester: | 0,2 g |
| Entmineralisiertes Wasser: | ad 100,0 g |

Herstellung:

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel III

Puder mit 0,1 % Wirkstoff

| | |
|---|---|
| Wirkstoff des Beispiels Nr. 4: | 0,1 g |
| Zinkoxid: | 10,0 g |
| Magnesiumoxid: | 10,0 g |
| Hochdisperses Siliciumdioxid: | 2,5 g |
| Magnesiumstearat: | 1,0 g |
| Talkum: | 76,4 g |

Herstellung

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

**Patentansprüche**

1. Diarylacetylene der Formel I

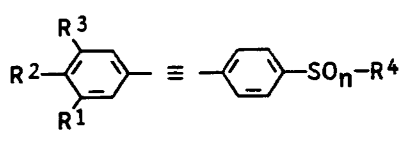

I

in der $R^1$ Wasserstoff oder eine $C_1$-$C_4$-Alkylgruppe bedeutet, $R^2$ und $R^3$ je ein Wasserstoffatom oder - wenn $R^1$ in der Bedeutung von Wasserstoff steht - unter Bildung eines Zyklus gemeinsam eine $-C(CH_3)_2$-B-$C(CH_3)_2$-Gruppe (mit B in der Bedeutung von $-CH_2CH_2$-oder $-CH(CH_3)$-) oder eine $-C(CH_3)(Z)CH_2CH_2O$-Gruppe (mit Z in der Bedeutung einer $C_1$-$C_2$-Alkylgruppe) darstellen oder
$R^2$ eine $C_1$-$C_3$-Alkoxygruppe und $R^3$ eine $C_1$-$C_{10}$-Alkylgruppe, die geradkettig, verzweigt oder (poly-)cyclisch sein kann, bedeuten oder - wenn $R^2$ für ein Wasserstoffatom steht - $R^1$ und $R^3$ Alkylreste in der oben angegebenen Bedeutung darstellen,
$R^4$ für einen $C_1$-$C_6$-Alkylrest steht und

n gleich 0, 1 oder 2 ist.

2. Diarylacetylene der Formel I gemäß Anspruch 1, wobei $R^2$ und $R^3$ unter Bildung eines Zyklus gemeinsam eine $-C(CH_3)_2-B-C(CH_3)_2$-Gruppe darstellen.

3. Diarylacetylene der Formel I gemäß Anspruch 1, wobei $R^1$ und $R^3$ $C_1$-$C_4$-Alkylgruppen darstellen.

4. Diarylacetylene der Formel I gemäß Anspruch 2, wobei n gleich 2 ist.

5. Diarylacetylene der Formel I gemäß Anspruch 1, wobei $R^3$ den Adamantylrest und $R^2$ eine $C_1$-$C_3$-Alkoxygruppe darstellt.

6. Verfahren zur Herstellung der Diarylacetylene der Formel I gemäß einem der Ansprüche 1 bis 5, dadurch gekennzeichnet, daß man

a) Stilbene der Formel II

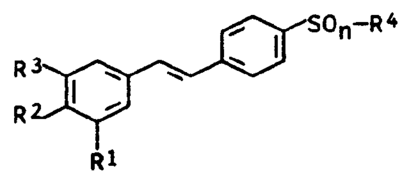

II

in der $R^1$ bis $R^4$ und n die im Anspruch 1 angegebene Bedeutung haben, halogeniert und anschließend 2 Mole Halogenwasserstoff abspaltet, oder

b) ein α-Chlorbenzylphosphonat der Formel III,

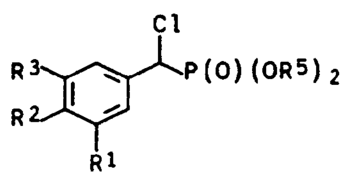

III

in der $R^1$ bis $R^3$ die oben angegebene Bedeutung haben und $R^5$ eine $C_{1-3}$-Alkylgruppe bedeutet, mit Aldehyden der Formel IV,

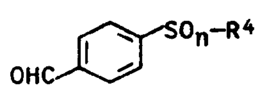

IV

mit $R^4$ und n in der im Anspruch 1 angegebenen Bedeutung umsetzt, oder

c) ein α-Chlorbenzylphosphonat der Formel V,

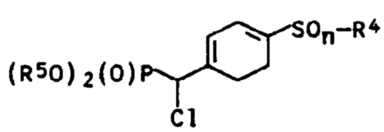

V

in der $R^4$, $R^5$ und n die oben angegebene Bedeutung haben, mit Aldehyden der Formel VI

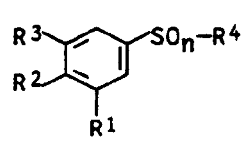

VI

worin $R^1$ bis $R^3$ die oben angegebene Bedeutung haben, umsetzt, oder
d) Monoarylacetylene der Formel VII,

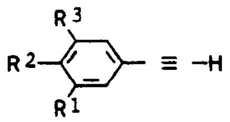

VII

worin $R^1$ bis $R^3$ die oben angebene Bedeutung haben, mit einem Arylhalogenid der Formel VIII

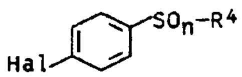

VIII

worin $R^4$ und n die oben angegebene Bedeutung zukommt und Hal Chlor, Brom oder Jod darstellt, in Gegenwart eines Katalysators und einer Base umsetzt, oder
e) Arylhalogenide der Formel IX

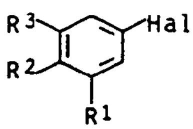

IX

worin $R^1$ bis $R^3$ und Hal die oben angegebene Bedeutung zukommt, mit einem Monoarylacetylen der Formel X

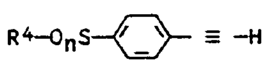

X

worin $R^4$ die oben angegebene Bedeutung besitzt, in Gegenwart eines Katalysators und einer Base umsetzt
und die so erhaltenen Verbindungen gegebenenfalls nach Standardmethoden in weitere Verbindungen der Formel I umwandelt.

7. Arzneimittel zur topischen Anwendung oder kosmetische Zubereitung, neben üblichen galenischen oder kosmetischen Hilfsmitteln als Wirkstoff enthaltend 0,001 bis 1 Gew.% eines Diarylacetylens der Formel I gemäß einem der Ansprüche 1 bis 5.

8. Arzneimittel zur systemischen Anwendung, das neben üblichen galenischen Hilfsmitteln als Wirkstoff pro Einzeldosis 0,1 bis 250 mg eines Diarylacetylens der Formel I gemäß einem der Ansprüche 1 bis 5 enthält.

9. Arzneimittel nach Anspruch 7 zur Behandlung von Akne oder Psoriasis oder anderen mit pathologisch veränderter Verhornung einhergehenden dermatologischen Erkrankungen sowie von Ekzemen, Warzen und Vitiligo.

10. Arzneimittel nach Anspruch 7 zur Behandlung von durch UV-Licht oder iatrogen bedingten Schädigungen der Haut.

## Claims

1. A diarylacetylene of the formula I

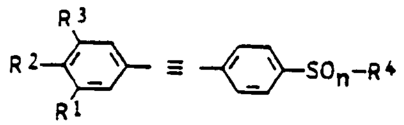

I

where $R^1$ is hydrogen or $C_1$-$C_4$-alkyl, $R^2$ and $R^3$ are each hydrogen or, when $R^1$ is hydrogen, together form a $-C(CH_3)_2$-B-$C(CH_3)_2$- group (where B is $-CH_2CH_2$- or $-CH(CH_3)$-) or a $-C(CH_3)$ (Z)$CH_2CH_2O$- group (where Z is $C_1$-$C_2$-alkyl) or $R^2$ is $C_1$-$C_3$-alkoxy and $R^3$ is $C_1$-$C_{10}$-alkyl which can be straight-chain, branched or (poly)cyclic, or, when $R^2$ is hydrogen, $R^1$ and $R^3$ are each alkyl as defined above,
$R^4$ is $C_1$-$C_6$-alkyl and
n is 0, 1 or 2.

**2.** A diarylacetylene of the formula I as claimed in claim 1, where $R^2$ and $R^3$ together form a -C $(CH_3)_2$-B-$C(CH_3)_2$- group.

**3.** A diarylacetylene of the formula I as claimed in claim 1, where $R^1$ and $R^3$ are each $C_1$-$C_4$-alkyl.

**4.** A diarylacetylene of the formula I as claimed in claim 2, where n is 2.

**5.** A diarylacetylene of the formula I as claimed in claim 1, where $R^3$ is adamantyl and $R^2$ is $C_1$-$C_3$-alkoxy.

**6.** A process for the preparation of a diarylacetylene of the formula I as claimed in any of claims 1 to 5, which comprises
a) halogenating a stilbene of the formula II

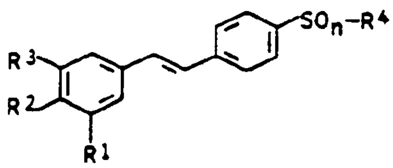

II

where $R^1$ to $R^4$ and n have the meanings specified in claim 1, and subsequently eliminating 2 moles of hydrogen halide, or
b) reacting an α-chlorobenzylphosphonate of the formula III

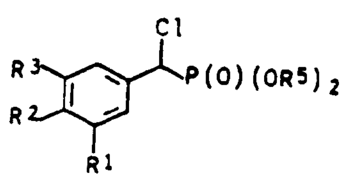

III

where $R^1$ to $R^3$ have the abovementioned meanings, and $R^5$ is $C_1$-$C_3$-alkyl, with an aldehyde of the formula IV

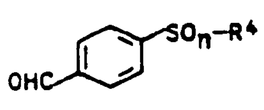

IV

where $R^4$ and n have the meanings specified in claim 1, or
c) reacting an α-chlorobenzylphosphonate of the formula V

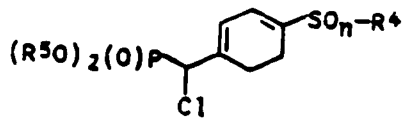

V

where $R^4$, $R^5$ and n have the abovementioned meanings, with an aldehyde of the formula VI

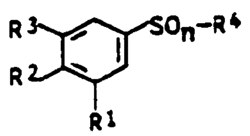

VI

where $R^1$ to $R^3$ have the abovementioned meanings, or
d) reacting a monoarylacetylene of the formula VII

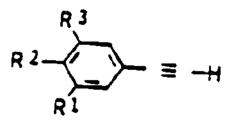

VII

where $R^1$ to $R^3$ have the abovementioned meanings, with an aryl halide of the formula VIII

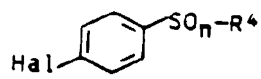

VIII

where $R^4$ and n have the abovementioned meanings, and Hal is chlorine, bromine or iodine, in the presence of a catalyst and of a base, or
e) reacting an aryl halide of the formula IX

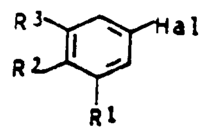

IX

where $R^1$ to $R^3$ and Hal have the abovementioned meanings, with a monoarylacetylene of the formula X

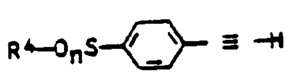

X

where $R^4$ has the abovementioned meanings, in the presence of a catalyst and of a base,
and converting the resulting compound where appropriate by standard methods into another compound of the formula I.

7. A drug for topical use or cosmetic preparation which contains as active compound from 0.001 to 1% by weight of a diarylacetylene of the formula I as claimed in any of claims 1 to 5, in addition to conventional pharmaceutical or cosmetic auxiliaries.

8. A drug for systemic use which contains as active compound per single dose from 0.1 to 250 mg of a diarylacetylene of the formula I as claimed in any of claims 1 to 5, in addition to conventional pharmaceutical auxiliaries.

9. A drug as claimed in claim 7 for the treatment of acne or psoriasis or other dermatological disorders associated with pathological cornification and of eczema, warts and vitiligo.

10. A drug as claimed in claim 7 for the treatment of skin damage which is iatrogenic or caused by UV light.

## Revendications

1. Diarylacétylènes de formule I

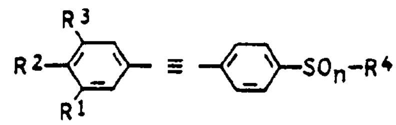

I

dans laquelle $R^1$ représente l'hydrogène ou un groupe alkyle en C1-C4, $R^2$ et $R^3$ représentent chacun un atome d'hydrogène ou bien - lorsque R1 représente l'hydrogène - ils représentent ensemble, avec formation d'un cycle, un groupe $-C(CH_3)_2-B-C(CH_3)_2-$ (dans lequel B représente $-CH_2CH_2-$ ou $-CH(CH_3)-$) ou un groupe $-C(CH_3)(Z)CH_2CH_2O-$ (dans lequel Z représente un groupe alkyle en C1-C2), ou bien $R^2$ représente un groupe alcoxy en C1-C3 et $R^3$ un groupe alkyle en C1-C10 qui peut être à chaîne droite, ramifiée ou (poly)cyclique, ou bien - lorsque $R^2$ représente un atome d'hydrogène - $R^1$ et $R^3$ représentent des groupes alkyle tels que définis ci-desus,
$R^4$ représente un groupe alkyle en C1-C6, et
n est égale à 0, 1 ou 2.

2. Diarylacéthylènes de formule I selon la revendication 1, dans laquelle $R^2$ et $R^3$ représentent ensemble, avec formation d'un cycle, un groupe $-C(CH_3)_2-B-$ $C(CH_3)_2-$.

3. Diarylacétylènes de formule I selon la revendication 1, dans laquelle $R^1$ et $R^3$ représentent des groupes alkyle en C1-C4.

4. Diarylacétylènes de formule I selon la revendication 2, dans laquelle n est égal à 2.

5. Diarylacétylènes de formule I selon la revendication 1, dans laquelle $R^3$ représente le groupe adamantyle et $R^2$ un groupe alcoxy en C1-C3.

6. Procédé de préparation des diarylacétylènes de formule I selon une des revendications 1 à 5, caractérisé en ce que :
a) on halogène des stilbènes de formule II

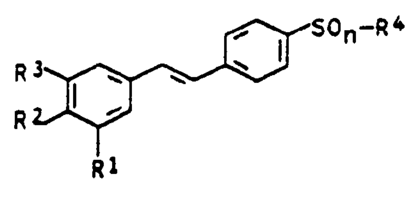

II

dans laquelle $R^1$ à $R^4$ et n ont les significations indiquées dans la revendication 1, puis on scinde 2 mol d'halogènure d'hydrogène, ou bien
b) on fait réagir un $\alpha$-chlorobenzylphosphonate de formule III

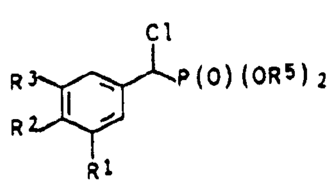

III

dans laquelle $R^1$ à $R^3$ ont les significations indiquées ci-dessus et $R^5$ représente un groupe alkyle en C1-C3, avec des aldéhydes de formule IV

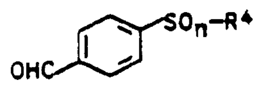

IV

dans laquelle $R^4$ et n ont les significations indiquées dans la revendication 1, ou bien
c) on fait réagir un α-chlorobenzylphosphonate de formule V

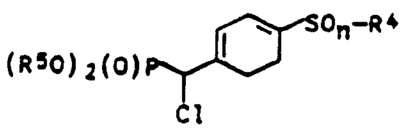

V

dans laquelle $R^4$, $R^5$ et n ont les significations indiquées ci-dessus, avec des aldéhydes de formule VI

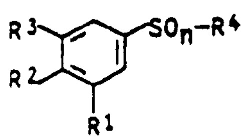

VI

dans laquelle $R^1$ à $R^3$ ont les significations indiquées ci-dessus, ou bien
d) on fait réagir en présence d'un catalyseur et d'une base des monoarylacétylènes de formule VII

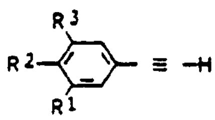

VII

dans laquelle $R^1$ à $R^3$ ont les significations indiquées ci-dessus, avec un halogénure d'aryle de formule VIII

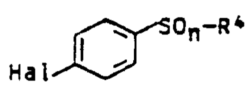

VIII

dans laquelle $R^4$ et n ont les significations indiquées ci-dessus et Hal représente le chlore, le brome ou l'iode, ou bien
e) on fait réagir en présence d'un catalyseur et d'une base des halogénures d'aryle de formule IX

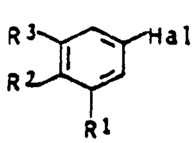

IX

dans laquelle $R^1$ à $R^3$ et Hal ont les significations indiquées ci-dessus, avec un monoarylacétylène de formule X

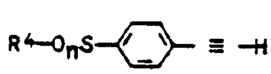

X

dans laquelle $R^4$ a les significations indiquées ci-dessus,
et le cas échéant on convertit les composés obtenus, par des modes opératoires classiques, en d'autres

composés de formule I.

7. Médicament pour l'application topique ou composition cosmétique contenant en tant que substance active, avec des produits auxiliaires galéniques ou cosmétiques usuels, de 0,001 à 1 % en poids d'un diarylacétylène de formule I selon les revendications 1 à 5.

8. Médicament pour l'application systémique contenant, en tant que substance active et par dose unitaire, avec des produits auxiliaires usuels, de 0,1 à 250 mg d'un diarylacétylène de formule I selon l'une des revendications 1 à 5.

9. Médicament selon la revendication 7, pour le traitement de l'acné ou du psoriasis ou d'autres maladies dermatologiques s'accompagnant d'une kératinisation pathologique, ainsi que de l'eczéma, des verrues et du vitiligo.

10. Médicament selon la revendication 7, pour le traitement des lésions de la peau provoquées par la lumières ultraviolette ou d'origine iatrogène.